# EUROPEAN PATENT APPLICATION

(11) **EP 3 971 289 A1**
(43) Date of publication of application: **23.03.2022**
(21) Application number: 20196950.8
(22) Date of filing: 18.09.2020
(51) Int. Cl.: C12N 15/10, C12Q 1/6806, C12Q 1/70

(54) **KIT FOR COLLECTING SAMPLES**

(71) Applicant: Procomcure Biotech GmbH, 5303 Thalgau (AT)
(72) Inventor: Wallerstorfer, Daniel, 5204 Straßwalchen (AT); Önder, Kamil, 5201 Seekirchen am Wallersee (AT)
(74) Representative: dompatent von Kreisler Selting Werner - Partnerschaft von Patent- und Rechtsanwälten mbB

(57) **Abstract**

The present invention refers to a receptable for body fluids wherein inside the receptable an coronavirus deactivating composition is present in solid form, use of said receptable for the collection of saliva, a kit for collecting saliva samples comprising said receptable, the use of said kit for collecting saliva, especially saliva comprising viral RNA and a method for the detection of RNA or DNA. The solid coronavirus deactivating composition can contain guanidinium salt, tris(hydroxymethyl)aminomethane and EDTA.

## Description

The present invention refers to a receptable for body samples wherein inside the receptable a coronavirus deactivating composition is present in solid form, use of said receptable for the collection of samples, a kit for collecting samples comprising said receptable, the use of said kit for collecting samples, especially samples comprising viral RNA and a method for the detection of RNA or DNA.

Pandemic situations are critical and challenging for the health systems worldwide. The impact of a pandemic can quickly overwhelm the public health and healthcare delivery systems throughout the world. Search capacity for staffing, availability of drugs and supplies, and alternate means to provide care must be included in detailed plans that are tested and drilled ahead of time. Accurate information on the disease must be made available to health-care staff and the public to reduce fear. Gathering of information and testing of people has become of outmost importance in order to control the pandemic and safely reopen the economy. This holds especially true for highly contagious diseases such as diseases caused by coronavirus. According to the centres of disease control and prevention, coronavirus disease 2019, or "COVID-19" is a respiratory illness caused by severe acute respiratory syndrome coronavirus 2, or "SARS-CoV-2". This illness spreads through respiratory droplets produced when an infected individual sneeze or coughed. Symptoms include fever, cough and shortness of breath. Severe complications include pneumonia, multi-organ failure, and in some cases death.
Viral tests, which detect active COVID-19 infections, are the primary type of testing although antibody tests, which indicate past infections based on the presence of COVID-19 antibodies, are also available.
There are several reasons why testing people for active COVID-19 infections is important. SARS-CoV-2 is especially contagious because this is the first time that this particular coronavirus is being transmitted among humans. Without a vaccine or herd-immunity, the only way to prevent transmission of COVID-19 is by separating people who are infected from those who are not. People who receive a positive diagnosis should self-isolate to prevent further spread of COVID-19. If people know with certainty that they are infected with COVID-19, they are more likely to take proper precautions, including isolation, social-distancing, and mask-wearing. This information is also beneficial to those around the infected individuals, as they can avoid contact altogether or exercise more caution when near the infected person.
This is especially important since there is evidence that a significant number of people with COVID-19 are asymptomatic, meaning they never display any of the common signs of illness or have symptoms to such a mild degree that COVID-19 can be mistaken for another illness, like the common cold. Studies have indicated that anywhere from 40% to 75% of people with COVID-19 are asymptomatic. Additionally, the more people there are being treated for confirmed cases of COVID-19 the more data doctors, epidemiologists, and researchers have to better understand the disease, its effects, and its possible treatments.
Understanding who is infected with COVID-19 and where outbreaks are likely is a critical factor in managing the pandemic from preparing hospitals for potential surges to safely reopening business, schools, churches and other gathering places safely. As noted above, because of how contagious the virus is, people must know with relative certainty whether they are currently infected with the disease before having contact with others at work, social gatherings, or in other public spaces. Otherwise, they run the risk of spreading the disease throughout their communities.
During widespread lockdowns public-health experts warned that lifting stay-at-home orders and returning to regular work and social habits without widespread testing would likely lead to a surge in infections. These experts emphasized that effective widespread testing to prevent outbreaks is the key part of getting the economy up and running again. However, there are challenges to increase COVID-19 testing to the recommended levels. Testing has become challenging due to a lack of supplies and backlogs at the laboratories that process diagnostic test. Furthermore, clinics and laboratories need to provide skilled people for collecting suitable body samples. In comparison with classic sampling via a nasal swap, collecting a saliva sample is easier, not invasive and not unpleasant for the person to be tested. Thanks to the user-friendly aspects, the use of saliva samples can be an advantage, for example, during a SARS-CoV-2 outbreak in a school where children need to be tested. There is also less risk for healthcare professionals when taking a saliva sample because patients can take it themselves and there is less risk of coughing or sneezing then when taking a sample with a swap.

In view of the problems associated with the testing systems there is a demand for a sample collection system which allows the collection of samples from persons to be tested at home. This, however, requires a reliable and safe handling of the collection procedure which is a challenging task. Furthermore, the collection of samples at home further require a save and stable storage of the collected samples in a manner that the samples can be safely transported by postal services to laboratories doing the sample analysis. In particular it is important that DNA and RNA, such as viral RNA are not destroyed or damaged. The above-mentioned problems have been solved by the embodiments as reflected in the present invention.

An objective of the present invention is to provide a receptable for the collection of body samples from a human or animal body which allows save and stable storage of the collected samples and which provides sufficient safety to the person to be tested or the person doing the collection. Thus, the receptable for body samples of the present invention is suitable for the self-collection of body samples by the test person itself, e.g. at home.

One embodiment of the present invention is a receptable for body samples wherein inside of said receptable an coronavirus deactivating composition is present in solid form.

Receptables for body samples within the meaning of the present invention are generally considered to be in-vitro diagnostic medical devices. The receptables are specifically suitable for the containment and preservation of specimens derived from the human body, especially, but not limited to, body fluids, for the purpose of in-vitro diagnostic examination. Preferably the receptable of the present invention is a sample tube or a vial. In one aspect of the invention the receptable may have a capacity for liquids of 0.5 ml to 1l, preferably ranging from 1 ml to 200 ml, especially ranging from 1.5 ml to 20 ml or 2 ml to 10 ml. In a further aspect the receptable has a sealable opening and is preferably a sealable sample tube.

In a further aspect the receptable can advantageously be liquid tight sealed more preferably air-tight sealed. Air-tight sealing or liquid-tight sealing is advantage for the transport of the samples in order to avoid contaminations. Sealable receptable which can easy and safely sealed by customer or in general tested persons are especially preferred.

Therefore, in one further aspect of the present invention the receptable of the invention has a cap, preferable a screw cap.
The receptable of the present invention may in general consist of any material which is suitable for the contact with body fluids and/or biological material, in particular body samples from the human or animal body. Non-limiting examples are glass or organic polymers. Organic polymers are preferred due to the improved break and shock resistance which allows persons doing the sample collection at home even a safe handling.
Especially good results could be achieved with a receptable which comprises or consists of polystyrene or of polyolefins, preferably polyolefins such as polypropylene and/or polyethylene.
In one preferred aspect the receptable is essentially consisting of an organic polymer having a melting point higher than 120 °C, preferably higher than 140 °C and most preferably a melting point higher than 150 °C, e. g. between 150 and 200°C. A higher melting point is advantage for the receptable of the present invention since in a preferred aspect the coronavirus deactivating composition is precipitated on the inner surface of the receptable by evaporating water from a composition comprising the coronavirus deactivating composition, preferably at higher temperatures such as higher than 80°C or higher than 90°C.

In one aspect of the invention the receptable consists essentially of high density polyolefins. In a further aspect the material of the receptable may be different from the material of the sealing such as the cap.
In one aspect the receptable of the invention comprises or consist essentially of polypropylene and the cap comprises or is consisting essentially of polyethylene. Preferably the receptable has the form of a collection tube.
The receptable of the present invention has an inner surface which is suitable to be contacted with body fluids or body samples in general and which is in contact with the collected body sample when used and filled. Inside the receptable of the invention is a solid coronavirus deactivating composition. The solid coronavirus deactivating composition is water soluble at 20°C.
Usually, the composition can be added into the receptable in solid form, preferably in powder form. In one aspect the inner surface of the receptable is at least partly coated with the coronavirus deactivating composition. Advantageously, the composition adheres at least partly, preferably completely to the inner surface of the receptable.
In a specially preferred embodiment of the invention the coronavirus deactivating composition is deposited on the inner surface of the receptable by precipitation. Precipitation is done according to a preferred aspect by evaporating the solvent, preferably water, of a solution or dispersion comprising the coronavirus deactivating composition in the receptable of the invention.
It has been found that a receptable which contains a solid coronavirus composition is safer for subjects to be tested. Especially when the receptable is used in a kit for sample collection, preferably a kit comprising a second receptable with a liquid formulation such as a gargling formulation, it can be avoided that the person in use mistakenly takes the receptable comprising the coronavirus composition as the gargling formulation.

The deactivation of coronaviruses is known in the literature (Evaluation of Chemical Protocols for Inactivating SARS-CoV-2 Infectious Samples; Viruses 2020, 12, 624*).*
It has been found that coronaviruses, especially SARS-CoV-2, can be deactivated by chaotropes. Certain embodiments described herein may include at least one chaotrope in the composition for substantially stable storage of nucleic acid and/or polypeptide molecules in a biological sample. A number of chaotropes or chaotropic agents are known in the art that disrupt the secondary, tertiary and/or quartenary structures of biological macromolecules such as polypeptides, proteins and nucleic acids, including DNA and RNA. Non-limiting examples of such chaotropes as are contemplated for use in certain of the presently disclosed embodiments include guanidinium salts, guanidinium hydrochloride, guanidinium thiocyanate, potassium thiocyanate, sodium thiocyanate and urea. Certain contemplated embodiments, including those that may relate to specific types of biological samples, expressly exclude the presence of a chaotrope when a chelating agent is also present, and in particular of a chaotrope at a concentration sufficient to denature a protein, polypeptide or nucleic acid molecule, while certain other contemplated embodiments are not so limited. Certain embodiments, including but not limited contemplate inclusion of a chaotrope at a concentration of about 0.05, 0.1, 0.5, 1.0, 1.2, 1.4, 1.6, 1.8, 2.0, 2.2, 2.4, 2.6, 2.8, 3.0, 3.2, 3.4, 3.4, 3.6, 3.8 or 4.0 M, where "about" may be understood to represent quantitative variation that may be more or less than the recited amount by less than 50%, more preferably less than 40%, more preferably less than 30%, and more preferably less than 20%, 15%, 10% or 5%.

A major cause of nucleic acid instability in biological samples is the presence of deoxyribonucleases and ribonucleases. Deoxyribonucleases and ribonucleases are enzymes that break down DNA or RNA, respectively. Their main source in the digestive tract is secretions of the pancreas, although these enzymes may also be present in secretions and cells of the salivary gland and buccal mucosa. In addition, microorganisms resident in the mouth or from recently ingested foods may release deoxyribonucleases or ribonucleases. Over time, the nucleic acid within a biological sample (e.g., saliva) stored in water would be expected to degrade or break down.
Guanidinium salts, especially guanidinium thiocyanate and/or guanidinium hydrochloride are also known to inhibit deoxyribonucleases and ribonucleases (Methods in Enzymology; Volume 502, 2012, Pages 273-290).

According to a preferred embodiment the coronavirus deactivating composition comprises or consists of a guanidinium salt in solid form.

In a further aspect of the present invention the coronavirus deactivating composition may comprise a solidified buffer.

Non-limiting examples of suitable buffering agents include sodium cyclohexane diaminetetraacetate (CDTA), N,N-bis(2-hydroxyethyl)-2-aminoethanesulfonic acid (BES), 4-(2-Hydroxyethyl)piperazine-1-ethanesulfonic acid (HEPES), acetic acid or acetate (e.g. sodium acetate), citric acid or citrate, malic acid, phthalic acid, succinic acid, histidine , pyrophosphoric acid, maleic acid, cacodylic acid, BB'-Dimethylglutaric acid, carbonic acid or carbonate, 5(4) Hydroxymethylimidazole, glycerol 2-phosphoric acid, ethylenediamine, imidazole, arsenic acid, phosphoric acid or phosphate, sodium acetate, 2 : 4 : 6 - collidine, 5(4)-methylimidazole,N-ethylmorpholine, triethanolamine, diethylbarbitu ric acid, tris (hydroxymethyl ) aminomethane (Tris), 3-(N Morpholino) propanesulfonic acid; 4-morpholinepropanesulfonic acid (MOPS), 2 - morpholinoethanesulfonic acid ( MES), piperazine-1,4-bis (2-ethane sulfonic acid)(PIPES), N-[tris( hydroxymethyl)methyl)-2 aminoethanesulfonic acid (TES), 4-(2-Hydroxyethyl)piperazine-1-propanesulfonic acid (EPPS), N-(2 acetamido)-2-aminoethanesulfonic acid (ACES), or combinations thereof . Other examples include phosphate, carbonate, ethylenediamine or imidazole buffers. Additional non-limiting examples of suitable buffering agents include buffering agents having a pKa at 25°C of from about 4.7 to about 8.0.

It has been found advantage and improves the storage stability of the collected samples if the coronavirus deactivating composition comprises tris(hydroxymethyl)aminomethane and/or ethylenediaminetetraacetic acid.

In a preferred embodiment the coronavirus deactivating composition comprises tris(hydroxymethyl)aminomethane and a guanidinium salt in a molar ratio ranging from 1:1000 to 1:10, more preferably 1:500 to 1:20, most preferably 1:150 to 1:50.

In a further preferred embodiment, the coronavirus deactivating composition comprises ethylenediaminetetraacetic acid and a guanidinium salt in a molar ratio ranging from 1:1000 to 1:20, preferably 1:600 to 1:50, even more preferably 1:300 to 1:100.

In a further preferred aspect of the present invention the coronavirus deactivating composition comprises tris(hydroxymethyl)aminomethane and ethylenediaminetetraacetic acid in a molar ratio ranging from 20:1 to 0.5:1, preferably 10:1 to 0.8:1 and most preferably 5:1 to 1:1.

In a further aspect of the present invention the guanidinium salt is selected from the group consisting of guanidinium thiocyanate, guanidinium hydrochloride, guanidinium iso-thiocyanate and mixtures thereof.

Most preferably the guanidinium salt is guanidinium thiocyanate and/or guanidinium hydrochloride.

The receptable of the present invention is suitable for the collection of body samples.
In certain embodiments the body sample is selected from blood, urine, serum, serosal fluid, plasma, lymph, cerebrospinal fluid, saliva, mucosal secretion, vaginal fluid, ascites fluid, pleural fluid, pericardial fluid, peritoneal fluid, feces, body tissues and abdominal fluid.
The body fluid may dissolve the solidified coronavirus deactivating composition in the receptable of the invention. The volume of the receptable is usually adapted to provide a sufficient concentration of the corona deactivating composition when completely filled with body fluid. Alternatively, for non-liquid body samples the samples may be diluted with sterile water or sterile solutions which do not affect the sample stability.
According to a further aspect of the present invention the body fluid is saliva.
The term "saliva", as used herein, refers to the secretion, or combination of secretions, from any of the salivary glands, including the parotid, submaxillary and sublingual glands, optionally mixed with the secretions from the numerous small labial, buccal, and palatal glands that line the mouth.

Advantages to the subject of providing a saliva sample or nasal, anterior nasal and/or nasopharyngeal sample, rather than a blood sample as a source of ribonucleic acid, include that subjects typically prefer avoiding the discomfort, pain and apprehension associated with phlebotomy. Additionally, and although use of a pin - prick to obtain a drop of blood is sufficient to recover a useable amount of DNA and RNA. Saliva, sputum, nasal, anterior nasal and/or nasopharyngeal samples have a further advantage of not requiring specialized personnel for collection, thereby reducing cost where mass sample collection is being carried out (e.g., during a epidemic/pandemic). However, it will be clear to the skilled worker that while saliva is one source of RNA, other bodily fluids and body samples, including blood can be used. The present invention is not intended to be limited to the collection and storage of RNA obtained from sputum, saliva, nasal, anterior nasal and/or nasopharyngeal samples. To collect saliva from a subject it is preferred that the mouth be rinsed before sampling. Food particles can introduce foreign RNA and saliva transferred by kissing can be a source of foreign human RNA or viral RNA. The mouth can be rinsed with about 50 ml of water by vigorous swishing or by brushing with a toothbrush without toothpaste. Unstimulated saliva is usually of the mucinous type and is secreted at a slow rate. Stimulated saliva (anticipation of tasty food, sweet or sour candy) is of the serous (watery) type and secreted at a faster rate. After rinsing of the mouth and waiting about 5 minutes for the mouth to clear of water, the subject may spit a volume (for example, about 1 - 2 ml) of saliva, preferably stimulated saliva, into the receptable of the present invention. Saliva flow can conveniently be stimulated with a few grains/pinch of table sugar placed on top of the tongue, or any other such saliva - stimulatory substance that does not interfere with RNA stability or subsequent amplification. Saliva may also be obtained from subjects such as infants, young children and people with disabilities and/or illness that may be unable to directly spit into a collection device. In this instance, an implement (e.g., a swab etc.) is used to collect saliva. Saliva may also be obtained from non - human animals such as livestock, companion animals and the like, which may be unable or unwilling to directly spit into a collection device. In this instance, an implement (e.g., a swab etc.) can be used to collect saliva. To collect anterior nasal or nasopharyngeal samples from a subject, a variety of implements may be used. Mucosal cells can be scraped using rigid or flexible brushes, swabs, or plastic/wood scrapers and cells may be flushed from the nasal cavity by introducing a liquid (e.g., saline) and recovering the liquid. For example, a rigid swab/brush can be placed in the anterior of the nose and a flexible swab/brush into the posterior nasopharyngeal cavity and used to collect mucosal secretions and to gently rub off cells from the mucosal membrane. Samples collected with said liquid and/or implement(s) can be delivered into the receptable of the invention.

A further embodiment of the present invention is the use of the receptable of the present invention for the collection of body samples.

It has been found that infections by the coronavirus may lead to severe acute respiratory syndromes and that gargling provides a sufficient virus load for the detection of viral RNA, e.g. via PCR technics. On the other hand the present invention is not limited to the collection of saliva samples or body fluid samples. Even body samples which are not completely liquid, such as feces can be collected. For samples which are not or not entirely in liquid form a diluting solution may be used when filling into the receptable of the invention. Further, in some preferred embodiments of the invention mixing the samples with liquid formulations which are provided in a kit, such as a gargling composition or a diluting composition can be advantage for the sample collection process.

Therefore, another embodiment of the present invention is a kit for collecting body samples comprising
a) a first receptable for body samples according to the present invention,
b) a device to collect the body samples; and
c) optionally a filling device, which is preferably a funnel.

The kit is suitable of collecting a human body samples and stabilising it for transport. The downstream process is the extraction of DNA or RNA followed by PCR analysis. It has been found that the kit of the invention is excellent for the collection of saliva samples and stabilises the sample for it to be transported at ambient temperatures through normal postal a courier services.

The kit of the invention may additionally comprise or consist of one or more of the following components:
a container for the transport of the receptable of the invention filled with the body sample, e.g. a saliva sample or a feces sample. The container may be a sealable plastic bag;
labelling sticker to label the samples; and absorbable material.

The device to collect body samples may be a second receptable for a gargling composition or any other device suitable to take a body sample. Non-limiting examples are spatula, swabs, syringes, cannulas, a second receptable containing a diluting composition such as sterile water, saline, etc.

The kit consists of a receptable of the invention and preferably filling device such as a funnel to collect human gargling solution or body fluid or diluted body samples. This kit allows for the at-home-collection of cells from the back of the throat and of saliva. The sample is stabilised, protein is denatured and prepared for transport at ambient temperatures. The receptable ensures that the samples are not contaminated during transport.
The samples may then be used in specifically equipped facilities to extract the DNA or RNA from the stabilised cells or saliva. The downstream application of the sample is an analysis of the DNA or RNA. The purpose of this analysis may be medical in nature for virology or have non-medical lifestyle ancestry and nutrition purposes.
In one preferred aspect the kit of the invention comprises a receptable of the invention in form of a sample tube with a sealable opening, preferably a cap.
The user removes the cap of the collection tube and places the filling device, preferably the funnel into the opening of the tube. The user than opens a second receptable comprising a gargling composition and takes gargling composition, preferably water, into his mouth. The gargling composition is used for gargling at the back of the throat for a sufficient time, e.g. 10 seconds. The user transfers the liquid into the first receptable using the funnel. The funnel is discarded and the tube closed with the enclosed cap. The tube is returned to the plastic back for transport. The user would than send the samples to the facility for analysis. Alternatively, other body samples can be collected as is known to the person skilled in the technical field.

According to a preferred embodiment of the present invention the kit comprises a receptable for body samples which is a sealable sample tube comprising or consisting of polypropylene or polyethylene or polystyrene.

In a further embodiment of the present invention the kit for collecting samples according to the present invention may comprise a second receptable a sample tube or an ampule, wherein said second receptable preferably comprises or is consisting of polypropylene or polyethylene or polystyrene.

In one aspect of the present invention the gargling composition, which is present in the second receptable, comprises or consists of water or buffered saline.

A further embodiment of the present invention is the use of a kit of the present invention for collecting body samples such as saliva, especially body samples comprising viral RNA, especially RNA from coronavirus such as RNA from SARS-CoV-2 virus.

A further embodiment of the present invention is a method for the detection of RNA or DNA comprising the following steps:
i) providing a body sample,
ii) filling the body sample into a receptable of the invention; and
iii) analysing the sample.

In a preferred embodiment of the invention the body sample is a sample of saliva which is obtained by gargling with a gargling composition.
Methods of the invention are conveniently practiced by providing the compositions used in such method in the form of a kit, e.g. the kit of the invention. Such a kit preferably contains a receptable of the invention and appropriate collecting devices such as swabs to facilitate sample collection. At least one type of positive control or standard may be provided that can be a nucleic acid (DNA or RNA) template for demonstrating the suitability of the sample for the detection of a target gene or nucleic acid sequence (e.g. transcript). Desirably, the container facilitates collection in the field, without the requirement of a clinic or hospital, and is sized to be mailed to a collection site and/or an analysis site.

In a further preferred embodiment, the present invention refers to the extraction of viral RNA, especially RNA from coronavirus such as RNA from SARS-CoV-2 virus.

Preferably the detection of RNA or DNA is the detection of viral RNA, especially RNA from coronavirus such as RNA from SARS-CoV-2 virus.

In a specially preferred embodiment of the method according to the present invention the sample is analyzed with PCR techniques, such as reverse transcription polymerase chain reaction (RT-PCR) or RT-qPCR or RNA/DNA sequencing or RNA/DNA hybridization.

### Examples:

The receptable of the invention was prepared in that a sample tube consisting of polypropylene was filled with 1.5 ml of an aqueous composition with a concentration of 4M guanidinium thiocyanate, 55 mM tris(hydroxymethyl)aminomethane (Tris) and ethylenediaminetetraacetic acid (EDTA). The solution was incubated for 15h at 90°C. The sample tube with the solidified coronavirus deactivating composition was equipped with a screw cap consisting of polyethylene.

The receptable is intended for sample stabilization for downstream genetic analyses. Genetic analyses can be performed on DNA or RNA. Since RNA is considered significantly less stable than DNA, the stability validation was performed on RNA. Hence, it is assumed, that when RNA is stable, DNA will be even more stable. In addition, Viral RNA is considered even more unstable and so this validation explores 2 aspects of stability:
- Human RNA stability over time measuring RNAse-P RNA in the samples
- Viral (SARS-CoV-2) RNA over time

### Sample preparation

The aim of this experiment was to assess the stability of human and viral RNA over extended periods of time at 0 °C and at 45 °C. The typical time, in which a sample is taken and transported to the laboratory is less than 24h, but may be as slow as 72h. In order to evaluate the stability of these samples, the following experiment was set up:
Saliva samples from 4 individuals was collected with the kit of the invention comprising the above-mentioned receptable of the invention and a second receptable consisting of a sample tube containing 1 ml water. The test individuals used the gargling solution and gargled for 10 seconds. The gargling samples were subsequently collected. These individuals were previously tested for the presence of SARS-CoV-2 viral infection and found to be negative. The samples were split into 4 equal aliquots. Each aliquot was infused with real SARS-CoV-2 virus from a clinical sample previously analysed to be positive and carrying a high viral load. The CT-Value of the positive sample was 24.27 indicating a rough viral load of 250 000 viral copies per reaction. As the receptable of the invention should be able to preserve lower viral loads, the negative saliva samples were spiked with the positive viral material at a dilution o 1:500. This result in a sample that has 500 times less viral material than a strongly infectious individual, roughly 500 copies per reaction. (The detection limit of down to 2.5 copies is determined at a later stage in this application).

### 1. SAMPLE STABILITY OF HUMAN RNA

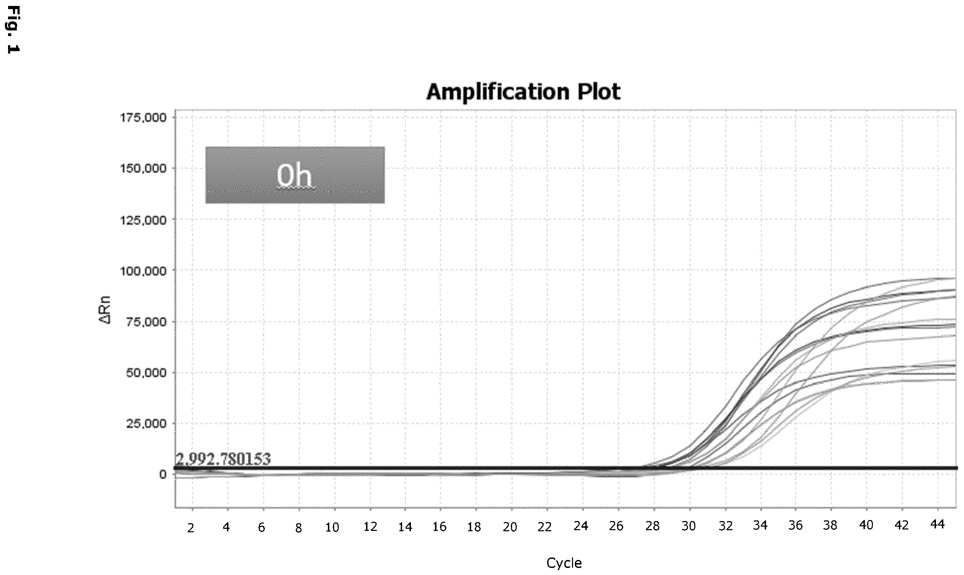

The presence of human RNA was measured at all time points in quadruplicate. The mean of the quadruplicate was taken and used for calculations. All amplification curves were displayed as an exponential rtPCR amplification curve and the CT value was determined. The curves are reflected in Fig. 1.

| **Mean HEC CT** | |
|---|---|
| Individual 1 | 30.46 |
| Individual 2 | 28.28 |
| Individual 3 | 31.98 |
| Individual 4 | 27.55 |
| Positive sample pool 1:10 diluted | 29.69 |

| | |
|---|---|
| AVERAGE CT: 0h @ 0°C, CT= 29.59 | |

### RESULT

Human RNA was roughly equally abundant in all of the samples (as was to be expected) as dilutions were done in negative human saliva samples.

The NTC, sample without human material showed no amplification as was to be expected.

### 2. SAMPLE STABILITY OF HUMAN RNA

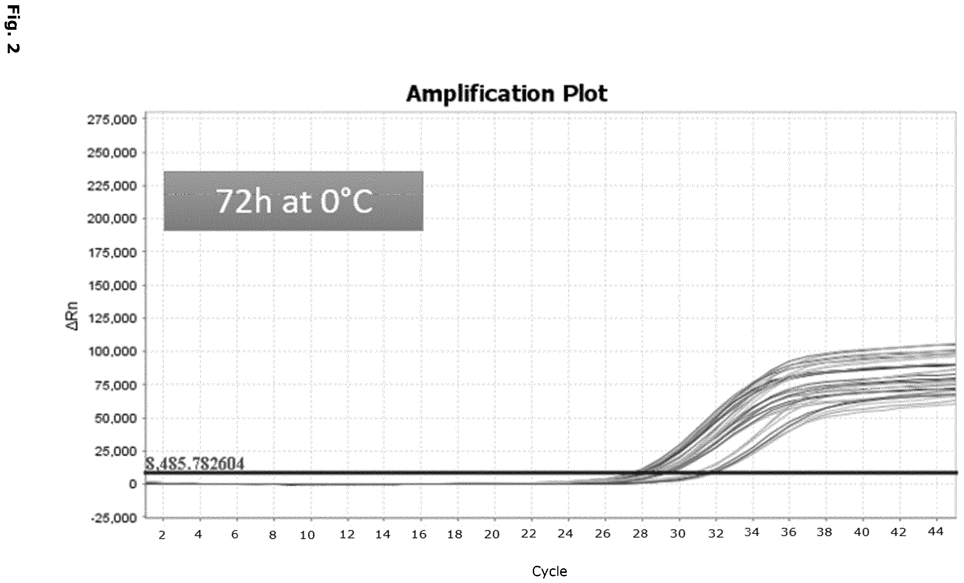

The presence of human RNA was measured at all time points in quadruplicate. The mean of the quadruplicate was taken and used for calculations. All amplification curves were displayed as an exponential rtPCR amplification curve and the CT value was determined. The curves are reflected in Fig. 2.

| **Mean HEC CT** | |
|---|---|
| Individual 1 | 31.22 |
| Individual 2 | 28.20 |
| Individual 3 | 28.92 |
| Individual 4 | 27.81 |
| Positive sample pool 1:10 diluted | 29.92 |

| | |
|---|---|
| AVERAGE CT: 0h @ 0°C, CT= 29.59 72h @ 0°C, CT= 29.22 | |

### RESULT

Human RNA was also stable at 45°C. There was no increase in the CT-Value, which is a measurement of degradation.

### 3. SAMPLE STABILITY OF HUMAN RNA

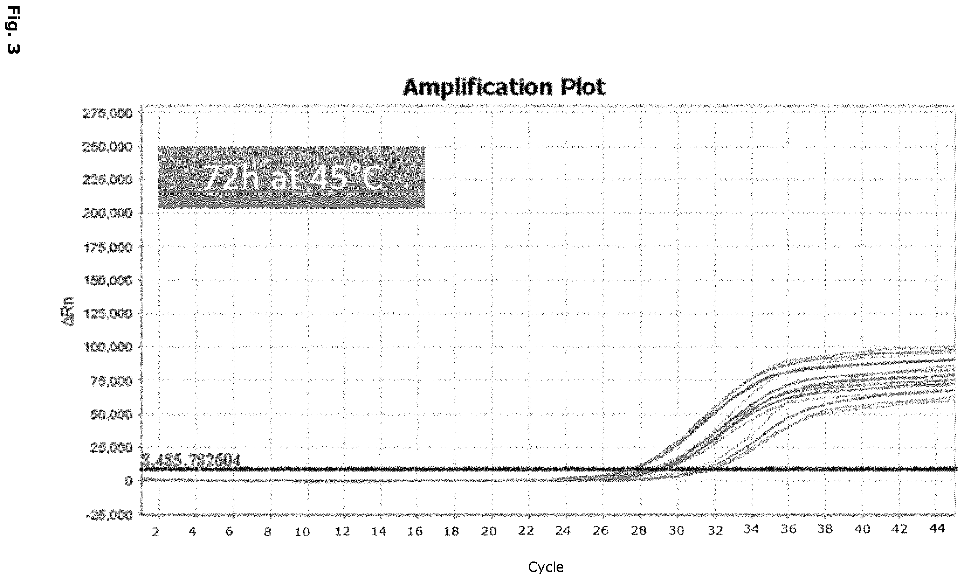

The presence of human RNA was measured at all time points in quadruplicate. The mean of the quadruplicate was taken and used for calculations. All amplification curves were displayed as an exponential rtPCR amplification curve and the CT value was determined. The curves are reflected in Fig. 3.

| **Mean HEC CT** | |
|---|---|
| Individual 1 | 31.45 |
| Individual 2 | 28.81 |
| Individual 3 | 29.02 |
| Individual 4 | 27.54 |
| Positive sample pool 1:10 diluted | 30.92 |

| | |
|---|---|
| AVERAGE CT: 0h @ 0°C, CT= 29.59 72h @ 0°C, CT= 29.22 72h @ 45°C, CT= 29.55 | |

### RESULT

Human RNA was also stable at 45°C. There was no increase in the CT-Value, which is a measurement of degradation.

### 4. SAMPLE STABILITY OF HUMAN RNA

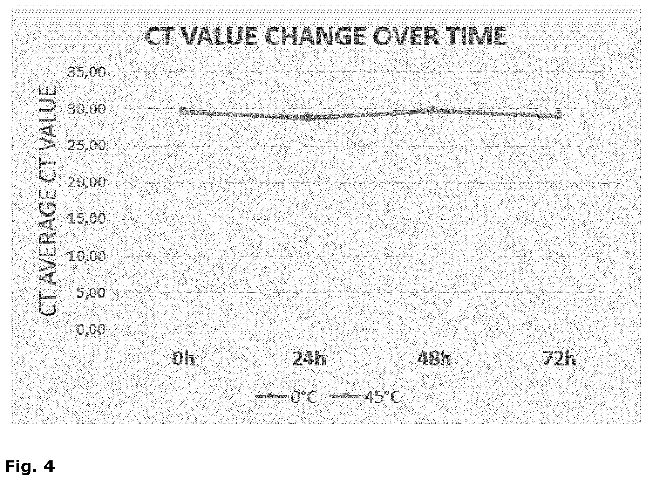

The presence of human RNA was measured at all time points in quadruplicate. The mean of the quadruplicate was taken and used for calculations. All amplification curves were displayed as an exponential rtPCR amplification curve and the CT value was determined. The curves are reflected in Fig. 4.

| **HUMAN CONTROL (Average CT)** | **Start** | **0°C 24h** | **0°C 48h** | **0°C 72h** | **45°C 24h** | **45°C 48h** | **45°C 72h** |
|---|---|---|---|---|---|---|---|
| INDIVIDUAL 1 | 30.46 | 28.89 | 32.13 | 31.22 | 30.66 | 32.16 | 31.45 |
| INDIVIDUAL 2 | 28.28 | 28.38 | 29.03 | 28.20 | 28.88 | 29.14 | 28.81 |
| INDIVIDUAL 3 | 31.98 | 29.05 | 29.92 | 28.92 | 28.85 | 29.88 | 29.02 |
| INDIVIDUAL 4 | 27.55 | 28.36 | 27.99 | 27.81 | 27.70 | 27.96 | 27.54 |
| **AVERAGE CT** | **29.57** | **28.67** | **29.77** | **29.04** | **29.02** | **29.78** | **29.20** |

### RESULTS

The CT-value was stable for both temperatures showing a high degree of stability. A temperature of 45°C simulates an accelerated age at room temperature of a factor 4.5. This means, that a period of 72h at this temperature is the equivalent of 72x4.5= 324h or 13.5 days. Thus, human RNA is stable for a minimum of 2 weeks at room temperatures.

### 5. SAMPLE STABILITY OF VIRAL RNA

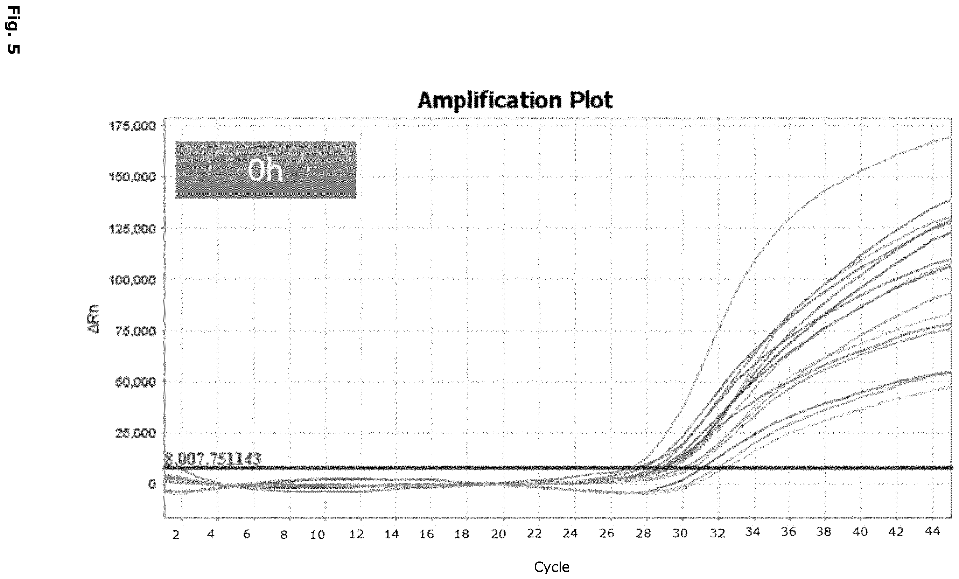

The presence of viral RNA was measured at all time points in triplicate. The mean of the triplicate was taken and used for calculations. All curves were displayed as an exponential rtPCR amplification curve and the CT value was determined. The curves are reflected in Fig. 5.

| **Mean SARS-CoV-2 CT** | |
|---|---|
| Individual 1 | 29.28 |
| Individual 2 | 29.19 |
| Individual 3 | 30.92 |
| Individual 4 | 28.36 |
| Positive sample pool 1:10 diluted | 24.27 |

| | |
|---|---|
| AVERAGE CT: 0h @ 0°C, CT= 29.44 | |

### 6. SAMPLE STABILITY OF VIRAL RNA

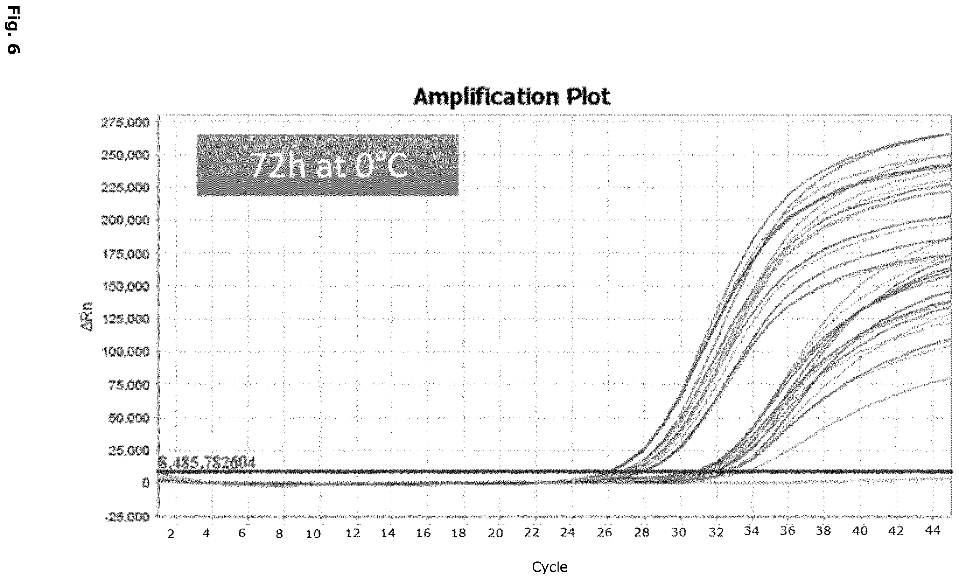

The presence of viral RNA was measured at all time points in triplicate. The mean of the triplicate was taken and used for calculations. All curves were displayed as an exponential rtPCR amplification curve and the CT value was determined. The curves are reflected in Fig. 6.

| **Mean SARS-CoV-2 CT** | |
|---|---|
| Individual 1 | 27.59 |
| Individual 2 | 26.82 |
| Individual 3 | 27.40 |
| Individual 4 | 26.06 |
| Positive sample pool 1:10 diluted | 23.26 |

| | |
|---|---|
| AVERAGE CT: 0h @ 0°C, CT= 29.44 72h @ 0°C, CT= 26.97 | |

### RESULT

VIRAL RNA was very stable at 0°C with no observable degradation.

### 7. SAMPLE STABILITY OF VIRAL RNA

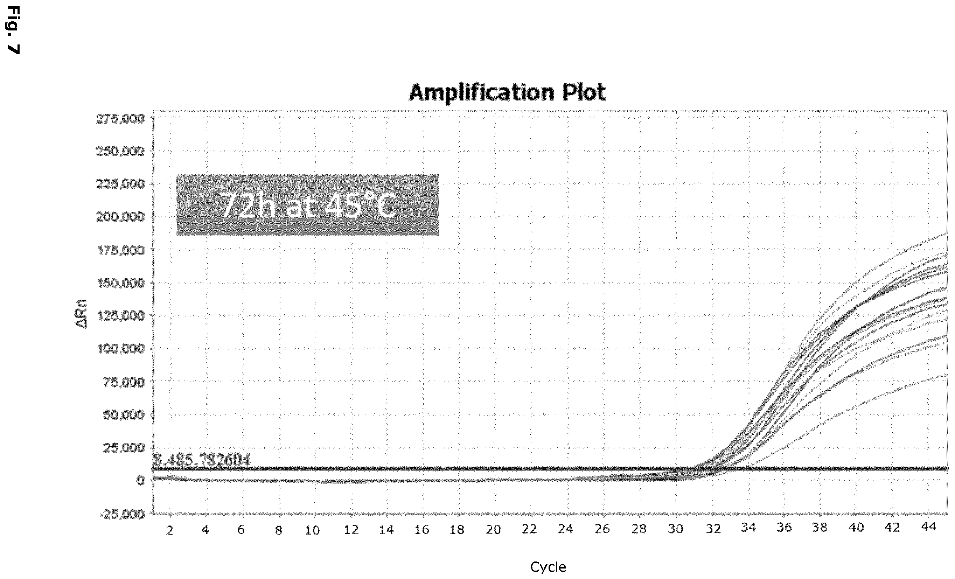

The presence of viral RNA was measured at all time points in triplicate. The mean of the triplicate was taken and used for calculations. All curves were displayed as an exponential rtPCR amplification curve and the CT value was determined. The curves are reflected in Fig. 7.

| **Mean SARS-CoV-2 CT** | |
|---|---|
| Individual 1 | 32.64 |
| Individual 2 | 31.16 |
| Individual 3 | 31.15 |
| Individual 4 | 32.00 |
| Positive sample pool 1:10 diluted | 30.36 |

| | |
|---|---|
| AVERAGE CT: 0h @ 0°C, CT= 29.44 72h @ 0°C, CT= 26.97 72h @ 45°C, CT= 31.74 | |

### RESULT

VIRAL RNA was also stable at 45°C. There was only a marginal increase in the CT-Value, which is a measurement of degradation.

### 8. SAMPLE STABILITY OF VIRAL RNA

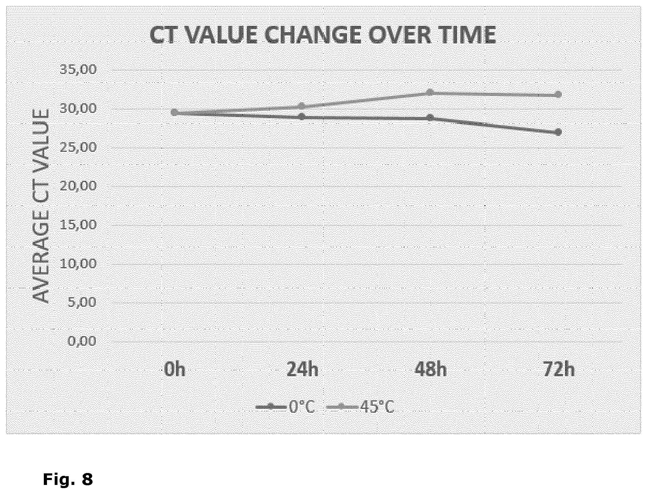

The presence of viral RNA was measured at all time points in triplicate. The mean of the triplicate was taken and used for calculations. All curves were displayed as an exponential rtPCR amplification curve and the CT value was determined. The curves are reflected in Fig. 8.

| **VIRAL RNA (Average CT)** | **Start** | **0°C 24h** | **0°C 48h** | **0°C 72h** | **45°C 24h** | **45°C 48h** | **45°C 72h** |
|---|---|---|---|---|---|---|---|
| INDIVIDUAL 1 | 29.28 | 30.92 | 29.62 | 27.59 | 30.11 | 33.07 | 32.64 |
| INDIVIDUAL 2 | 29.19 | 27.98 | 28.63 | 26.82 | 30.40 | 30.89 | 31.16 |
| INDIVIDUAL 3 | 30.92 | 28.71 | 29.11 | 27.40 | 30.21 | 31.78 | 31.15 |
| INDIVIDUAL 4 | 28.36 | 28.06 | 27.51 | 26.06 | 30.09 | 32.43 | 32.00 |
| **AVERAGE CT** | **29.44** | **28.92** | **28.72** | **26.97** | **30.20** | **32.04** | **31.74** |

### RESULTS

The CT-value was stable for both temperatures showing a high degree of stability. A temperature of 45°C simulates an accelerated age at room temperature of a factor 4.5. This means, that a period of 72h at this temperature is the equivalent of 72x4.5= 324h or 13.5 days. Thus, viral RNA is stable for a minimum of 2 weeks at room temperatures or 3 days at 45°C.

### 9. MULTIPLE FREEZE THAW CYCLES

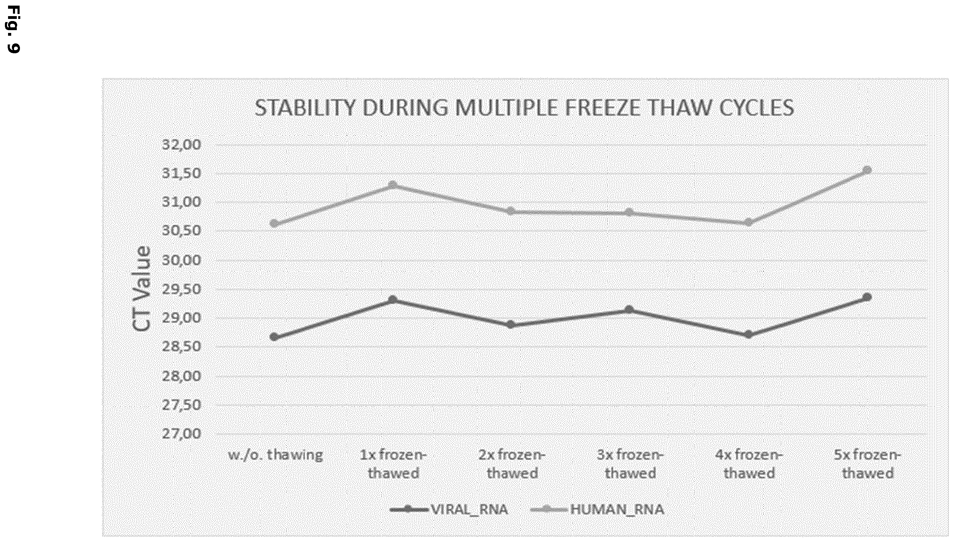

Freezing and thawing is considered to impact sample quality and lead to degradation of DNA and RNA. To assess if repeated freeze that cycles affect the sample stability, a sample was frozen at -20°C, then thawed to room temperature, measured and then re-frozen and rethawed and measured a total of 5 times. The curves are reflected in Fig. 9.

| Mean HEC CT | |
|---|---|
| w./o. thawing | 28.67 |
| 1x frozen-thawed | 29.30 |
| 2x frozen-thawed | 28.87 |
| 3x frozen-thawed | 29.14 |
| 4x frozen-thawed | 28.70 |
| 5x frozen-thawed | 29.34 |

| Mean SARS-CoV-2 CT | |
|---|---|
| w./o. thawing | 30.62 |
| 1x frozen-thawed | 31.30 |
| 2x frozen-thawed | 30.84 |
| 3x frozen-thawed | 30.81 |
| 4x frozen-thawed | 30.63 |
| 5x frozen-thawed | 31.55 |

### RESULT

Even 5 cycles of freezing and thawing do not affect stability of either viral or human RNA.

### 10. SAMPLE STABILITY OF VIRAL RNA

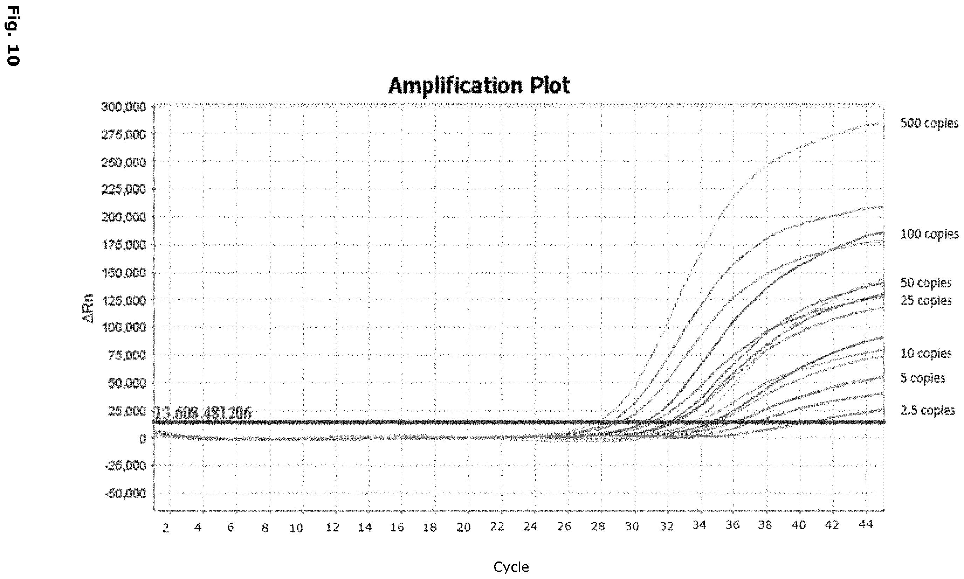

A serial dilution of viral copies was performed in samples of negative individuals. The estimated number of viral copies is shown in the diagram (Fig. 10).

### RESULT

The receptable of the invention allows accurate detection of down to 2.5 copies of viral RNA per reaction.

## Claims

1. Receptable for body samples **characterized in that** inside the receptable comprises a solid coronavirus deactivating composition.

2. Receptable according to claim 1 wherein the receptable contains a coronavirus deactivating composition comprising or consisting of a guanidinium salt in solid form.

3. Receptable according to claim 1 or 2 wherein the coronavirus deactivating composition is deposited on the inner surface of the receptable by precipitation.

4. Receptable according to one or more of the preceding claims wherein the coronavirus deactivating composition comprises a solidified buffer.

5. Receptable according to one or more of the preceding claims wherein the coronavirus deactivating composition comprises tris(hydroxymethyl)aminomethane and/or ethylenediaminetetraacetic acid.

6. Receptable according to one or more of the preceding claims wherein the coronavirus deactivating composition comprises tris(hydroxymethyl)aminomethane and guanidinium salt in a molar ratio ranging from 1:1000 to 1:10, preferably 1:500 to 1:20, more preferably 1:150 to 1:50.

7. Receptable according to one or more of the preceding claims wherein the coronavirus deactivating composition comprises ethylenediaminetetraacetic acid and guanidinium salt in a molar ratio ranging from 1:1000 to 1:20, preferably 1:600 to 1:50, more preferably 1:300 to 1:100.

8. Receptable according to one or more of the preceding claims wherein the coronavirus deactivating composition comprises tris(hydroxymethyl)aminomethane and ethylenediaminetetraacetic acid in a molar ratio ranging from 20:1 to 0.5:1, preferably 10:1 to 0.8:1, more preferably 5:1 to 1:1.

9. Receptable according to one or more of the preceding claims wherein the guanidinium salt is selected from the group consisting of guanidinium thiocyanate, guanidinium hydrochloride, guanidinium iso-thiocyanate and mixtures thereof.

10. Receptable according to one or more of the preceding claims wherein the guanidinium salt is guanidinium thiocyanate and/or guanidinium hydrochloride.

11. Receptable according to one or more of the preceding claims wherein the receptable is a sample tube, preferably a sample tube having a sealable opening, preferably a sealable sample tube.

12. Receptable according to one or more of the preceding claims wherein the body sample is selected from the group consisting of blood, urine, serum, serosal fluid, plasma, lymph, cerebrospinal fluid, saliva, mucosal secretion, vaginal fluid, ascites fluid, pleural fluid, pericardial fluid, peritoneal fluid, feces, tissue samples and abdominal fluid.

13. Use of the receptable according to one or more of the preceding claims for the collection of body samples, preferably body samples for the analysis of DNA and RNA.

14. Kit for collecting body samples comprising
a. a first receptable for body samples according to one or more of claims 1 to 12,
b. a device to collect the body samples; and
c. optionally a filling device, preferably a funnel.

15. Use of a kit according to one or more of claims 14 for collecting body samples, especially samples comprising viral RNA, especially RNA from coronavirus such as RNA from SARS-CoV-2 virus.

16. Method for the detection of RNA or DNA comprising the following steps:
i) Providing a body sample;
ii) Filling the body sample into a receptable according to one or more of claims 1 to 12; and
iii) Analysing the sample, preferably analyzing the sample with PCR techniques, such as reverse transcription polymerase chain reaction (RT-PCR) or RT-qPCR or RNA/DNA sequencing or RNA/DNA hybridization.
